# EUROPEAN PATENT SPECIFICATION

(11) **EP 1 838 229 B1**
(45) Date of publication and mention of the grant of the patent: **26.08.2009**
(21) Application number: 06718796.3
(22) Date of filing: 19.01.2006
(51) Int. Cl.: A61B 17/28, A61B 10/00, A61B 17/32

(54) **BIOPSY FORCEPS**
BIOPSIEZANGE
PINCES A BIOPSIE

(30) Priority: 20.01.2005 US 646104 P
(43) Date of publication of application: 03.10.2007
(73) Proprietor: Wilson-Cook Medical Inc., Winston-Salem, NC 27105-4191 (US)
(72) Inventor: SURTI, Vihar, C., Winston-Salem, NC 27106 (US)
(74) Representative: Garratt, Peter Douglas
(86) International application number: PCT/US2006/001775
(87) International publication number: WO 2006/078743

(56) References cited:
- EP-A- 0 738 501
- US-A- 5 669 927
- US-A- 5 899 919
- US-A- 5 913 874

## Description

### FIELD OF THE INVENTION

This invention generally relates to medical devices, and particularly to forceps used for obtaining biopsy samples.

### BACKGROUND

Physicians in many specialties commonly obtain biopsy samples from patients to determine the presence of tissue abnormalities, such as cancerous cells. Sometimes biopsies are taken without the need for an invasive procedure. For example,
physicians can take skin biopsies to test for melanoma. In many cases, however, a physician must access a biopsy location inside a patient's abdominal cavity, thoracic cavity, or gastro-intestinal system. For such procedures, physicians often use an endoscope to avoid more traumatic open surgery. Modem endoscopes are long, flexible instruments having a viewing system and a working channel through which a biopsy forceps can be passed.

Common endoscopic biopsy forceps are formed from a long shaft that extends between a proximal end and a distal end. The proximal end includes an actuator mechanism that a physician uses to control a small pair of biopsy jaws. The jaws are located at the distal end of the biopsy forceps, and are provided with teeth to cut,
shear, or tear away tissue samples. For biopsy forceps that are used through the working channel of an endoscope, the shaft of the biopsy forceps is longer than the endoscope so that the biopsy forceps jaws can extend out of the distal end of the endoscope and reach the target tissue. Shorter biopsy forceps are used to take biopsies from locations where introduction of the biopsy forceps through an endoscope is unnecessary. Reference is directed to EP-A-738 507 which shows an endoscopic operative instrument having an operative section which can include grasping members and a wire for manipulating the operative section, see preamble of claim 1.

Conventional biopsy forceps, however, have a number of drawbacks. For example, the actuator and jaw mechanisms are formed from numerous and miniscule components that require manual assembly. The manufacture of biopsy forceps is therefore expensive, difficult, and time consuming. There is thus a need for a biopsy forceps that resolves or improves upon any of these drawbacks.

### SUMMARY OF THE INVENTION

Accordingly, it is an object of the present invention to provide a medical device having features that resolve or improve upon one or more of the above-described drawbacks.

According to one aspect of the present invention, the foregoing object is obtained by providing a biopsy force having a sheath d an inner shaft slidably disposed within the sheath as specified in claim 1. The shaft has a longitudinal axis defined therethrough and a plurality of grasping members that are movable between an open configuration and a closed configuration. At least one of the plurality of grasping members is biased outwardly from the longitudinal axis when in the open configuration. At least one of the plurality of grasping members is unrestricted by the sheath when in the open configuration and is constrained by the sheath when in the closed configuration. The plurality of grasping members can be formed so that when they are in a closed configuration, they form a receptacle for retaining one or more biopsy samples. One or more of the grasping members may further be provided with a cutting edge to more easily remove a tissue sample.

According to another aspect of the present invention the shaft may be connectable to an electrocautery device. As a result, the shaft may be energized by the electrocautery device to electrosurgically cut the tissue.

### BRIEF DESCRIPTION OF THE DRAWINGS

Embodiments of the present invention will now be described by way of example with reference to the accompanying drawings (not to scale), in which:

Figure 1 is a perspective side view of a biopsy forceps head according to an embodiment of the present invention;

Figure 2 is a side view of a biopsy forceps head and a handle;

Figure 3A is a cross-sectional view of a biopsy forceps head according to an embodiment of the present invention showing an open configuration taken along line 3-3 of Figure 2;

Figure 3B is an end view of the embodiment shown in Figure 3A in a closed configuration having rectangularly shaped edges;

Figure 3C is an end view of an alternative embodiment of the embodiment shown in Figure 3A in a closed configuration having triangularly shaped edges;

Figure 4 is a flow-chart of a method of using a biopsy forceps according to one embodiment of the present invention;

Figure 5 is a side view of a biopsy forceps according to an embodiment of the present invention;

Figure 6 is a cross-sectional front view of a biopsy forceps head according to an embodiment of the present invention;

Figure 7 is an end view of a biopsy forceps head according to an embodiment of the present invention;

Figure 8A is an end view of an embodiment in the closed configuration having two grasping members with rectangularly shaped edges;

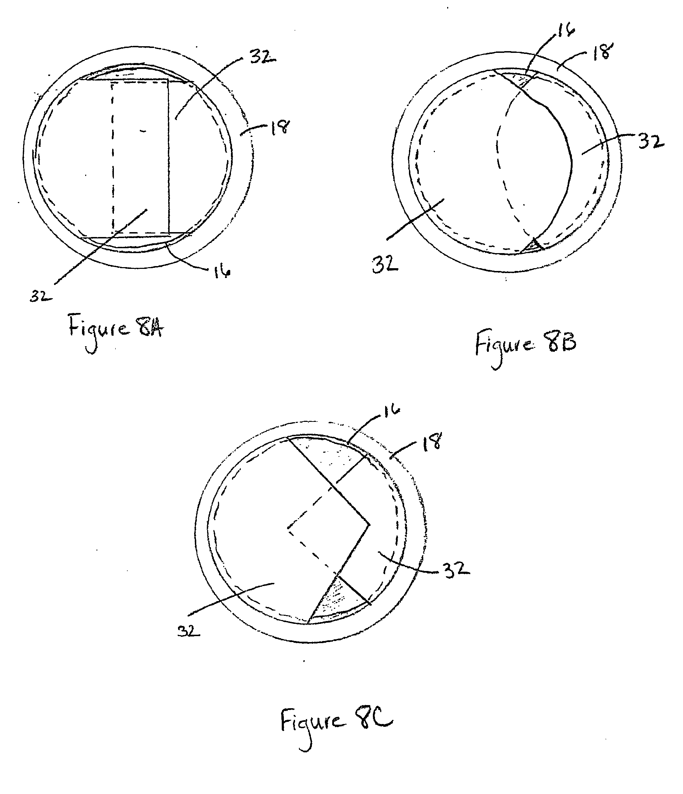

Figure 8A is an end view of an embodiment in the closed configuration having two grasping members with curved edges; and

Figure 8A is an end view of an embodiment in the closed configuration having two grasping members with triangularly shaped edges.

### DETAILED DESCRIPTION

The invention is described with reference to the drawings in which like elements are referred to by like numerals. The relationship and functioning of the various elements of this invention are better understood by the following detailed description. However, the embodiments of this invention as described below are by way of example only, and the invention is not limited to the embodiments illustrated in the drawings. It should also be understood that the drawings are not to scale and in certain instances details that are not necessary for an understanding of the present invention have been omitted, such as conventional details of fabrication and assembly. Moreover, it should be noted that the invention described herein includes methodologies that have a wide variety of applications.

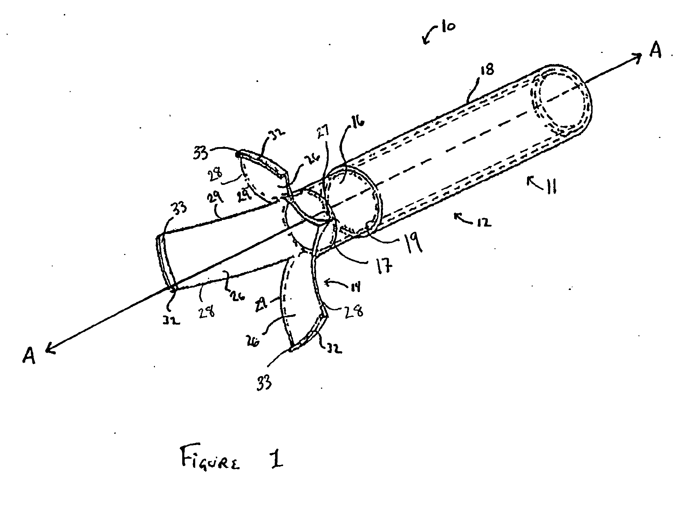

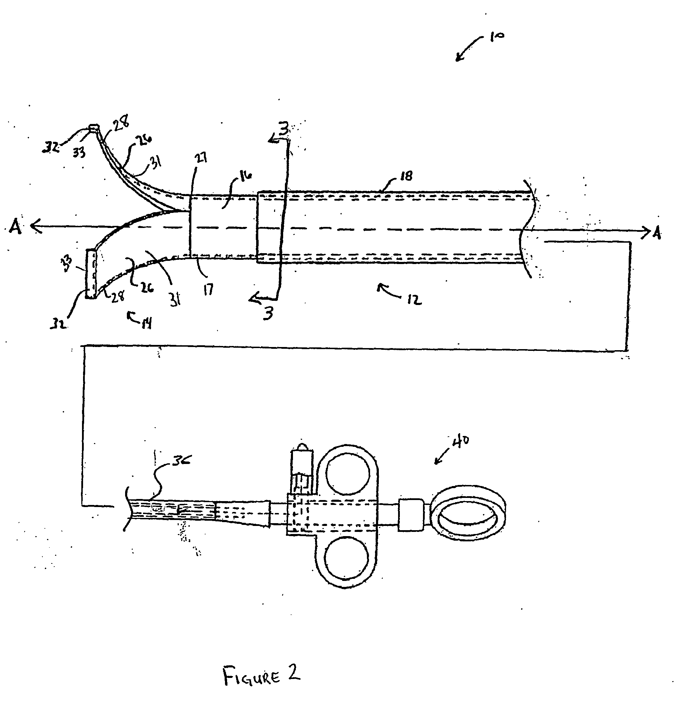

Referring to the drawings, Figures 1-3 depict an illustrative embodiment of the present invention. Generally, a medical device 10 is provided to take tissue samples for medical analysis. As illustrated in Figure 1, the medical device 10 includes a catheter 11 having a distal end 12. The distal end 12 includes an inner shaft 16 and grasping members 26 extending from a distal end 17 of the shaft 16. A longitudinal axis A is defmed through the shaft 16 as shown in Figures 1 and 2. The grasping members 26 are biased outwardly relative to the longitudinal axis A in an open configuration. The distal end 12 further includes an outer sheath 18 having a lumen 19 defined therein. In general, the shaft 16 is slidably received in the sheath 18 ( i.e., within the lumen 19). The shaft 16 may be slidable relative to the sheath 18 so that the shaft 16 may be retracted at least partially into the sheath 18 to cam the grasping members 26 into a closed, contracted configuration. A conventional handle 40 (shown in Figure 2) may be operably connected to a proximal end 36 of the catheter 11. The handle 40 may be used to control the movement of the shaft 16 relative to the sheath 18 and to control the movement of the grasping members 26 between the open configuration where the grasping members 26 are biased outwardly and the closed configuration where the grasping members 26 are cammed together.

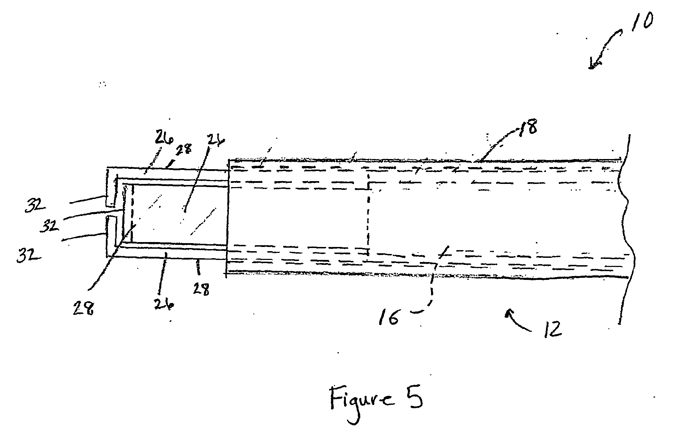

As illustrated in Figure 1, the distal end 12 of the catheter 11 includes the inner shaft 16, grasping members 26 and the sheath 18. In some embodiments, three grasping members 26 may extend from the shaft 16, although two, three, four, five or more grasping members 26 are possible, as will be understood by one skilled in the art. The grasping members 26 include a proximal portion 27 connected to the distal portion 17 of the shaft 16 and a distal portion 28 extending distally. The grasping members 26 are shown having a curvilinear profile in the open configuration where the distal portion 28 is biased away from the longitudinal axis A (Figure 1) and a substantially straight profile in the closed configuration (Figure 5). Alternative profiles for the grasping members are also possible including bent profiles and the like. In some embodiments, the grasping members 26 may extend from the distal portion 17 of the shaft 16 and be formed by unitary construction with the shaft 16 from a single elongate member such as a tube described below. The grasping members 26 can include an outer surface 31 that can be seen in Figure 2.

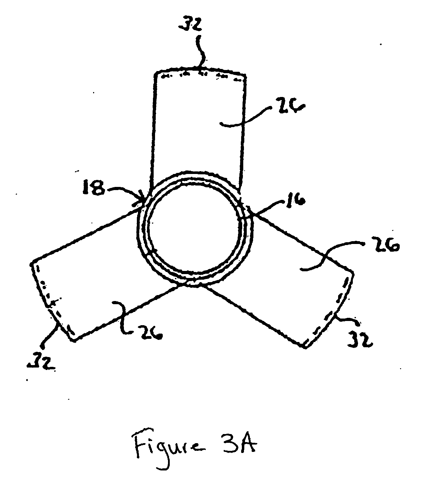

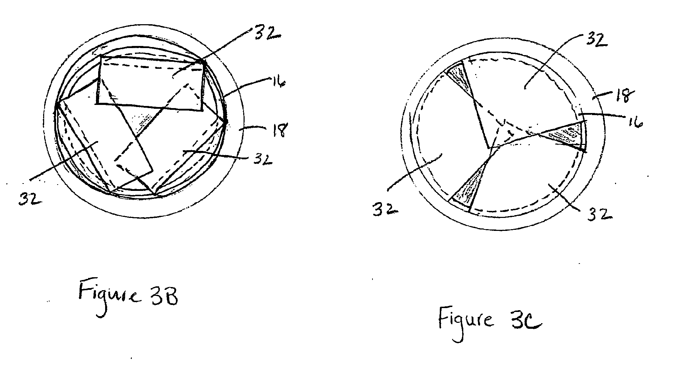

One or more of the grasping members 26 may be provided with a distal edge 32. In some embodiments, the distal edge 32 may be bent inwardly relative to the grasping member 26 and toward the longitudinal axis A, as shown in Figure 2. The distal edge 32 may be bent at a 90° angle with respect to the grasping member 26. The distal edge 32 may be adapted for shearing, grasping, tearing, or cutting tissue. The edge 32 may further include a blade portion 33 having a cutting surface. The edge 32 and blade portion 33 may be formed in any shape and configuration, including, but not limited to, a single blade or cutting surface, a crenate tooth configuration, straight, angular or curved. The distal edge 32, the blade 33, or both may be shaped to fit together so that the edges 32 or the blades 33 meet together at the distal portion 28 in the closed configuration. Figures 3A-C illustrate an embodiment having three grasping members. Figure 3A shows a cross-sectional view of the open configuration. Figure 3B shows an end view having distal edges 32 with a rectangularly shaped portions that overlap in the closed configuration. Figure 3C shows an alternatively shaped distal portion 28 having triangularly shaped distal edges 32 that overlap in the closed configuration. The embodiments shown in Figures 3B and 3C may also include the blade 33 on the distal edge 32. End views for alternative embodiments having two grasping members with alternatively shaped distal edges 32 are shown in Figures 8A-8C.

The distal edges 32 may be formed by removing material from the distal edge 32 to appropriately size and shape the edge 32 for fitting together, for example when the distal edge 32 is formed by bending a portion of the distal portion 28 of the grasping member 26 toward the longitudinal axis A. In embodiments having the blade 33, the blade 33 may be sized and shaped to fit together similarly to the distal edge 32. Alternatively, the distal edge 32 or the blade 33 or both may be formed by adding material to the distal portion 28 of the grasping members 26 in the desired size and shape.

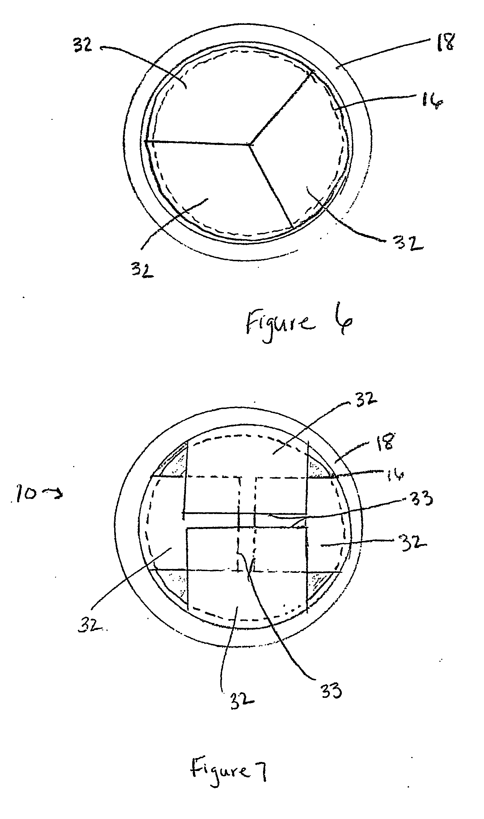

In some embodiments, the distal edges 32 may be sized and shaped to overlap each other as shown in Figures 5 and 7. For example, as shown in Figure 7 where the device 10 includes four grasping members 26, the opposite pairs of distal edges 32 may meet together with one pair extending further distally than the other pair so that the pairs overlap. In some embodiments, each distal edge 32 or blade 33 may overlap in the closed configuration, for example where three grasping members 26 are included with the device 10, the distal edges 32 may be triangularly shaped and overlap each other to form a generally triangularly shaped end of the receptacle, shown in end view in the closed configuration of Figure 3B. Any shape and size may be used to form the edge 32 and the blade 33 so that in the closed configuration, the sample may be held within the chamber formed by the grasping members 26 and the edges 32 that meet or overlap in the closed configuration. The edges 32 may be blunt or may include blades 33 to remove the tissue sample from the patient.

In some embodiments, the grasping members 26 may be curved around the longitudinal axis A to form a generally annular profile similar to shaft 16 when the shaft 16 is a cylindrically shaped cross section. The shaft 16 and the sheath 18 may also have alternatively shaped cross-sectional shapes, including polygonal and oval, and the like. In some embodiments, the distal portion 28 of the grasping members 26 may be flattened with the distal edges 32 being rectangularly shaped as shown in Figure 2. In some embodiments, the grasping members 26 may be relatively wide. The grasping members 26 may be used to capture a tissue sample within a chamber or receptacle formed by the grasping members 26 when the grasping members 26 are cammed together in the closed configuration. Longitudinal edges 29 of the grasping members 26 may be sized and shaped such that the longitudinal edges 29 of adjacent grasping members meet or are in proximity to form the chamber to hold the tissue sample when the device 10 is in the closed configuration. An example of the closed configuration is shown in Figure 5. The configurations described above may allow the edges 32 to firmly grasp and cut or tear the tissue to be biopsied and may prevent the tissue sample from dislodging from the device 10.

In some embodiments the grasping members 26 and the shaft 16 may be formed from resilient materials known to one of skill in the art. Any elastic material that can retain bending stresses and resiliently return to its preformed shape may be used. In some embodiments, metal may be used to form the device 10 or components thereof. Exemplary metals include stainless steel or an alloy having superelastic properties such as nitinol (NiTi). The shaft 16 and the grasping members 26 may be formed from a single piece of stainless steel tubing. A conventional programmable laser cutter can be programmed to laser-cut the tubing into the desired configuration. The laser cutter may be programmed to cut the desired shape repeatedly from a single length of tubing. The laser cutter may similarly be programmed to cut the shaft 16 to form any number of grasping members 26 (e.g., two, three, four, five, six, or more grasping members). Alternatively, the grasping members 26 may be welded or otherwise attached to the shaft 16 using techniques known to one skilled in the art. The grasping members 26 may be equally sized and shaped, or the grasping members 26 may be differently sized and shaped, for example, alternating between wider and narrower grasping members 26 or longer and shorter pairs of grasping members 26. The laser cutter may also be used to form the distal edges 32 and the blades 33 into any desired size and shape, for example, by removing a portion of material of the edges 32.

As illustrated in Figures 1-3, the sheath 18 may be slidably disposed over a portion of the shaft 16 to constrain the grasping members 26. The sheath 18 may slide relative to the shaft 16 to engage at least a portion of the outer surface 31 of the grasping members 26 to constrain the grasping members 26 in the closed configuration. In an exemplary embodiment, the sheath 18 may slide a distance between about 2 millimeters and 10 millimeters relative to the shaft 16, although a person of ordinary skill could alter the distance that the sheath 18 slides relative to the shaft 16. As the shaft 16 is retracted into or slides into the sheath 18-or alternatively as the sheath 18 slides forward over the shaft 16-the sheath 18 may cam the grasping members 26 into a closed configuration and constrain the grasping members 26. In some embodiments, the shaft 16, the sheath 18 or both may include a portion having a thin layer of lubricious material, such as polytetrafluoroethylene (PTFE) on surfaces that may contact each other, including the outer surface 31 of the grasping members 26. Sliding the sheath 18 in relation to the shaft 16 causes the grasping members 26 and the edges 32 to firmly grasp and cut, shear, or tear the tissue to be biopsied. The closed configuration with the sheath 18 slidably disposed over at least a portion of the grasping members 26 in the closed configuration may also allow for a narrower configuration for easy of delivery through a medical device, such as the working channel of an endoscope. The overall size and shape of the device 10 will depend on the location in which the device 10 will be used.

Operation of the biopsy forceps device 10 may be performed by any means known to one skilled in the art. For example, remote operation of the biopsy forceps device 10 may be controlled via a handle 40 at the proximal end 36 (Figure 2, showing an enlarged distal portion 12). As will become apparent to a person of ordinary skill, a wide variety of handle mechanisms could be used with the present invention. The handle 40 may be a thumb ring, a scissors-type handle, a pin vise, or any other conventional handle suitable for moving a sheath relative to a control wire or shaft. The handle 40 may also be connected to a control wire which is connected to the shaft 16 or the sheath 18. In general, the handle 40 is used to actuate the control wire, which in turn controls the movement of one of the shaft 16 or sheath 18 in relation to the other. In addition, the handle 40 may be used to maneuver the biopsy forceps device 10.

An electrical connector may be provided to energize the shaft 16 and grasping members 26 of the device 10. The electrical connector may conveniently form a male plug, which receives an electrical cord (sometimes called an 'active cord'). The electrical cord is connectable to a standard electrosurgical generator, such as those manufactured by Valleylab, Inc. (Boulder, CO). In use, a physician, via the generator, controls whether current is applied to the device 10, typically using a foot pedal to electrify the control wire and ablate tissue coming in contact with the stem, grasping members, or cutting edges. This allows a physician to cut or cauterize bleeding tissues with the shaft 16, grasping members 26, or cutting edges 32. The sheath 18 may be coated with insulating material, such as plastic or rubber, in some embodiments, as will be understood by one skilled in the art.

In some embodiments of the present invention, the biopsy forceps device 10 may be operably connected to an infusion source or a suction source. For example, in embodiments a suction device, such as a vacuum or a syringe may be connected to the shaft 16 to assist in tissue removal or general fluid removal around the biopsy site. The suction source may also be used to pull the biopsy sample back into the shaft 16 for removal or for taking multiple biopsy samples. Alternatively or in addition, the biopsy forceps device 10 may be operably connected to an infusion source such as a syringe or a pump to provide fluid to the biopsy site through the shaft 16. For example, saline, dye or medication may be infused through the shaft 16 to the biopsy site. Alternative lumens may be provided in the biopsy device 10 in addition to the shaft 16 to provide infusion or suction as needed. Any infusion device or suction source known to one skilled in the art may be operably connected to the biopsy device 10.

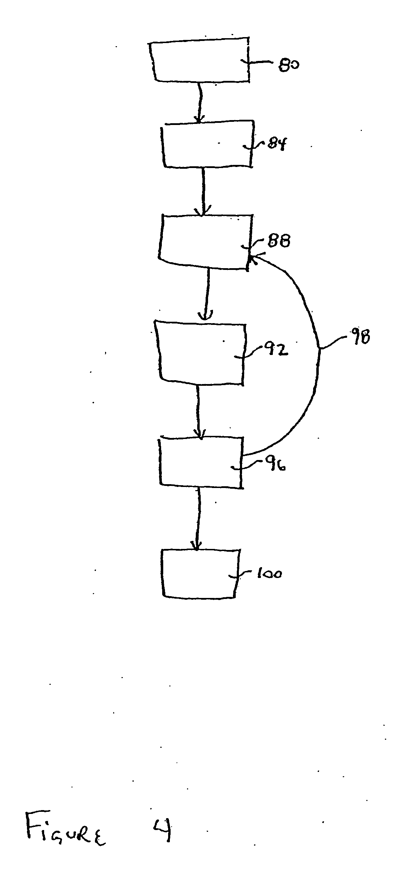

Figure 4 illustrates one method of utilizing the present invention to remove a biopsy sample from a target tissue. As illustrated in step 80, the target tissue is located, for example by using an endoscope. Once the target tissue is located, the biopsy forceps device 10 may be delivered to the target tissue, for example, by introducing the biopsy forceps device 10 through the working channel of the endoscope as illustrated in step 84. Alternatively the device 10 may be delivered to the tissue at the same time the tissue is being located. When the target tissue is located, the device 10 may be advanced toward the target biopsy tissue until the cutting edges 32 and/or the grasping members 26 are in contact with the target biopsy tissue, as shown in step 88. At this point in the procedure, a physician may manipulate the handle so as to slide either the shaft 16 or the sheath 18 to manipulate the grasping members 26 into position and then cam the grasping members 26 at least partially into the sheath 18 to move the grasping members 26 into the closed configuration, thereby grasping the targeted biopsy tissue, as shown in step 92. Thereafter, the physician may energize the grasping members 26 and shaft 16 with the energizing source, or simply tear, shear, or cut off a biopsy sample by pulling the device 10 away from the tissue, as shown in step 96. Optionally, the physician may take additional samples of the target tissue by repeating steps 88-96, as illustrated by step 98. Once the desired number of biopsy samples is taken, the physician can withdraw the device 10 and retrieve the biopsy sample for analysis, as shown in step 100.

Any other undisclosed or incidental details of the construction or composition of the various elements of the disclosed embodiment of the present invention are not believed to be critical to the achievement of the advantages of the present invention, so long as the elements possess the attributes needed for them to perform as disclosed. The selection of these and other details of construction are believed to be well within the ability of one of even rudimentary skills in this area, in view of the present disclosure. Illustrative embodiments of the present invention have been described in considerable detail for the purpose of disclosing a practical, operative structure whereby the invention may be practiced advantageously. The designs described herein are intended to be exemplary only. The novel characteristics of the invention may be incorporated in other structural forms without departing from the scope of the invention. Unless otherwise indicated, all ordinary words and terms used herein shall take their customary meaning as defined in The New Shorter Oxford English Dictionary, 1993 editi*on.* All technical terms shall take on their customary meaning as established by the appropriate technical discipline utilized by those normally skilled in that particular art area. All medical terms shall take their meaning as defined by Stedman's Medical Dictionary, 27th editi*on.*

## Claims

1. A biopsy forceps (10) comprising:
a sheath (18); and
an inner shaft (16) slidably disposed within the sheath and having a longitudinal axis defined therethrough, the shaft comprising a plurality of grasping members (26) connected to and extending distally from a distal portion of the shaft.
wherein at least one of the plurality of grasping members (26) is biased outwardly from the longitudinal axis and having a curvilinear profile when in an open configuration,
wherein the at least one of the plurality of grasping members (26) is unrestricted by the sheath when in the open configuration and is constrained by the sheath into a closed configuration by engaging the plurality of grasping members with the sheath, and
wherein the at least one of the plurality of grasping members includes a substantially straight profile in the closed configuration,
**characterised in that** one or more of the grasping members includes a cutting edge (32), and **in that** the shaft and the grasping members are integrally connected and formed by unitary construction from the same material.

2. The biopsy forceps of claim 1, wherein the cutting edge comprises a blade portion.

3. The biopsy forceps of claim 1 or claim 2, wherein the plurality of grasping members form a biopsy receptacle when in the closed configuration.

4. The biopsy forceps of any one of the preceding claims, wherein the shaft is formed from stainless steel.

5. The biopsy forceps of any one of the preceding claims, wherein the cutting edge is curvilinear.

6. The biopsy forceps of any one of the preceding claims, wherein the cutting edge comprises a jagged edge configured to tear tissue from a biopsy site.

7. The biopsy forceps of any one of the preceding claims, wherein the cutting edge is bent inward toward the longitudinal axis.

8. The biopsy forceps of any one of the preceding claims, wherein there are provided three or more grasping members.

9. The biopsy forceps of any one of the preceding claims, wherein a portion of the shaft is connectable to an electrocautery source to electrosurgically cut tissue.

## Patentansprüche

1. Biopsiezange (10), die folgendes umfasst:
eine Hülse (18) und einen Innenschaft (16), der gleitend in der Hülse angeordnet ist und eine durch ihn hindurch definierte Längsachse aufweist, wobei der Schaft eine Vielzahl von Greifelementen (26) umfasst, die mit einem distalen Abschnitt des Schafts verbunden sind und sich von diesem distal erstrecken, worin zumindest eines der Vielzahl von Greifelementen (26) von der Längsachse nach außen vorgespannt ist und in einer offenen Konfiguration ein krummliniges Profil aufweist, worin das zumindest eine der Vielzahl von Greifelementen (26) in der offenen Konfiguration nicht eingeschränkt ist und in einer geschlossenen Konfiguration von der Hülse eingezwängt wird, indem die Schleuse in die Vielzahl von Greifelementen eingreift, und worin das zumindest eine der Vielzahl von Greifelementen in der geschlossenen Konfiguration ein im Wesentlichen gerades Profil aufweist, **dadurch gekennzeichnet, dass** ein oder mehr der Greifelemente eine Schneidkante (32) aufweisen und dass der Schaft und die Greifelemente integral miteinander verbunden sind und einheitlich aus demselben Material geformt sind.

2. Biopsiezange nach Anspruch 1, worin die Schneidkante einen Klingenabschnitt umfasst.

3. Biopsiezange nach Anspruch 1 oder Anspruch 2, worin die Vielzahl von Greifelementen in der geschlossenen Konfiguration einen Aufnahmebehälter für die Biopsieprobe bilden.

4. Biopsiezange nach einem der vorhergehenden Ansprüche, worin der Schaft aus Edelstahl besteht.

5. Biopsiezange nach einem der vorhergehenden Ansprüche, worin die Schneidkante krummlinig ist.

6. Biopsiezange nach einem der vorhergehenden Ansprüche, worin die Schneidkante eine gezackte Kante umfasst, die Gewebe aus einer Biopsiestelle reißen kann.

7. Biopsiezange nach einem der vorhergehenden Ansprüche, worin die Schneidkante zur Längsachse hin nach innen gebogen ist.

8. Biopsiezange nach einem der vorhergehenden Ansprüche, worin drei oder mehr Greifelemente bereitgestellt sind.

9. Biopsiezange nach einem der vorhergehenden Ansprüche, worin ein Abschnitt des Schafts mit einer Elektrokauterisierungsquelle verbunden werden kann, um Gewebe elektrochirurgisch zu schneiden.

## Revendications

1. Pince à biopsie (10), comprenant :
une gaine (18); et
une tige interne (16) disposée de manière coulissante à l'intérieur de la gaine et ayant un axe longitudinal défini à travers elle, la tige comprenant une pluralité d'organes de préhension (26) connectés à une portion distale de la tige et s'étendant distalement depuis celle-ci,
au moins l'un de la pluralité d'organes de préhension (26) étant contraint vers l'extérieur de l'axe longitudinal et ayant un profil curviligne lorsqu'il est dans une position ouverte,
l'au moins un de la pluralité d'organes de préhension (26) n'étant pas restreint par la gaine lorsqu'il est dans la position ouverte et étant contraint par la gaine dans une configuration fermé par l'engagement de la pluralité d'organes de préhension avec la gaine, et
l'au moins un de la pluralité d'organes de préhension comportant un profil substantiellement droit dans la configuration fermée,
**caractérisée en ce qu'**un ou plusieurs des organes de préhension comportent un bord de coupe (32) et **en ce que** la tige et les organes de préhension sont connectés intégralement et formés par construction unitaire à partir du même matériau.

2. Pince à biopsie selon la revendication 1, dans laquelle le bord de coupe comprend une portion de lame.

3. Pince à biopsie selon la revendication 1 ou 2, dans laquelle la pluralité d'organes de préhension forme un réceptacle de biopsie lorsqu'ils sont dans la configuration fermée.

4. Pince à biopsie selon l'une quelconque des revendications précédentes, dans laquelle la tige est formée en acier inoxydable.

5. Pince à biopsie selon l'une quelconque des revendications précédentes, dans laquelle le bord de coupe est curviligne.

6. Pince à biopsie selon l'une quelconque des revendications précédentes, dans laquelle le bord de coupe comprend un bord dentelé configuré pour déchirer les tissus d'un site de biopsie.

7. Pince à biopsie selon l'une quelconque des revendications précédentes, dans laquelle le bord de coupe est courbé vers l'intérieur vers l'axe longitudinal.

8. Pince à biopsie selon l'une quelconque des revendications précédentes, dans laquelle il est prévu au moins trois organes de préhension.

9. Pince à biopsie selon l'une quelconque des revendications précédentes, dans laquelle une portion de la tige peut être connectée à une source d'électrocautérisation pour découper des tissus par voie électrochirurgicale.
